# EUROPEAN PATENT APPLICATION

(11) **EP 3 954 310 A1**
(43) Date of publication of application: **16.02.2022**
(21) Application number: 21193558.0
(22) Date of filing: 09.02.2018
(51) Int. Cl.: A61B 17/34, A61B 1/018, A61B 17/3205, A61B 18/14, A61B 17/00, A61B 18/00

(54) **SNARE INJECTION DEVICE**

(30) Priority: 10.02.2017 US 201762457689 P
(62) Divisional of application: 18751404.7
(71) Applicant: United States Endoscopy Group, Inc., Mentor, OH 44060 (US)
(72) Inventor: Russo, Jessica Lynn, Sagamore Hills, 44067 (US); Still, Rapheal, Richmond Heights, 44143 (US); Uspenski, Alex, Chardon, 44024 (US); Kaye, Christopher J., Eastlake, 44095 (US); John, Keith R., Chardon, 44024 (US); Pastor, Stephen T., Sagamore Hills, 44067 (US)
(74) Representative: Petraz, Gilberto Luigi

(57) **Abstract**

A medical device for performing an endoscopic procedure through an endoscope is provided. The device includes an elongated catheter having a first lumen and a second lumen; a needle system for injecting a target tissue within a body; and a base disposed at a proximal end of the catheter. The actuator tube is inserted through the base and the first lumen. A needle housing member is positioned between the needle and the first lumen when the needle is in a stored position. The needle housing member has a predetermined length so that the needle is disposed within the needle housing member when the needle is at the stored position. The needle housing member has a continuous inner surface and prevents the needle from penetrating the first lumen. The needle includes a tip, deflected towards a center axis of the needle. The first and second lumens have different deflected forces.

## Description

### CROSS-REFERENCES

The present application claims benefits and priority of U.S. Provisional Patent Application No. 62/457,689, filed on February 10, 2017.

### TECHNICAL FIELD

The subject matter relates to a medical device for performing endoscopic procedures and to an endoscopic device that can have a needle and a snare, or other endoscopic tools.

### BACKGROUND OF THE INVENTION

Endoscopes are well-known in the art and are commonly used for numerous medical procedures. One exemplary procedure is removing polyps, lesions or other types of targeted tissue from the gastrointestinal mucosal wall of a human subject. Various cauterization devices have been developed to remove polyps. These devices, however, sometimes cause serious thermal injury to the gastrointestinal wall, fail to remove the entire targeted tissue, or do not completely cauterize blood vessels which lead to excessive bleeding. Snare devices designed to encircle and remove polyps often do not capture all of the targeted tissue. Further, a physician may experience difficulty in securing the targeted tissue with the snare. Snaring only the minimal tissue required from the three layer wall, i.e., mucosa, submucosa, and muscularis, is also important. More specifically, to prevent complications, the muscularis tissue should be avoided all together in this type of procedure. In an effort to solve these and other problems, one technique used is a sub-mucosal lift polypectomy, which involves using a needle to inject the tissue with, for example, a saline solution, to lift the tissue to a more favorable position. This technique improves complete resection. The injected fluid separation also insulates the outer muscle from cautery or thermal injury.

Various other endoscopic procedures require a needle, including use of a needle for: washing a targeted work site; applying dyes for the purpose of highlighting diseased or abnormal tissue; injecting tattoo medium for post-resection surveillance purposes; and hemostatic injection therapy for post-polypectomy bleeding. In these and other procedures requiring a needle and a second endoscopic device, a physician must use two separate auxiliary instruments, and one at a time feed the devices in and out of the instrument channel of an endoscope, which increases the overall procedure time.

In certain situations, the needle may be combined with a second endoscopic device in one auxiliary instrument having a multiple lumen catheter. However, combining a needle with a second endoscopic device in a multiple lumen catheter can be problematic. For example, the needle may puncture the multiple lumen catheter or deploy beyond a desired length, injuring the patient or damaging the equipment.

### SUMMARY

The subject matter offers numerous advantageous features including a needle housing member for the needle lumen to prevent the needle from puncture through the needle lumen.

In another exemplary embodiment of the subject matter; the medical device includes a needle with a deflected, centered, or oriented tip towards the center axis of the needle. Alternatively, the needle has two-point deflected tip.

In another exemplary embodiment of the subject matter, the medical device includes a dual-lumen catheter member providing stiffness to the first lumen (i.e., the needle lumen) to help with the needle deployment, and flexibility to the second lumen (i.e., the snare lumen).

A medical device for performing an endoscopic procedure through an endoscope, the device comprising: a catheter having a first lumen and a second lumen; a retrieval system for retrieving a foreign object within a body, wherein the retrieval system comprises a retriever, and an actuating cable, wherein the cable is inserted through the second lumen, wherein the retriever is connected to a distal end of the actuator tube; and a base disposed at a proximal end of the catheter, wherein the actuator tube is inserted through the base and the second lumen; the retriever operably protrudes a predetermined distance out of a distal end of the catheter, wherein the first lumen and second lumen have different deflected forces.

In another exemplary embodiment of the subject matter, the medical device includes a catheter having a first lumen and a second lumen; a first device is inserted through the first lumen; and a second device is inserted through the second lumen; wherein the first and second lumens have different deflected forces.

### BRIEF DESCRIPTION OF THE DRAWINGS

FIG. 1 is a side view of a device according to an embodiment of the subject matter, wherein the device has a snare (shown deployed) and a needle;
FIG. 1a shows an alternative embodiment of the base assembly of FIG. 1;
FIG. 2 is an enlarged perspective view of the distal end of the device of FIG. 1, showing the snare and the needle deployed;
FIG. 3 is an embodiment of the catheter member of FIG. 1;
FIG. 4 is another embodiment of the catheter member of FIG. 1;
FIG. 5 is a cross sectional view of a distal end of the device of FIG. 1, showing the needle and snare retracted;
FIG. 6 is a cross sectional view of the distal end of the device of FIG. 1, showing the snare retracted and the needle fully deployed;
FIG. 6a is a cross-sectional view of the distal end of the device of FIG. 1, showing the snare retracted and the needle fully deployed where the needle includes integral stop;
FIG. 7 is a side view of an embodiment of the needle of the FIG. 1;
FIG. 8 is a front view of the needle of FIG. 7;
FIG. 9 is a side view of another embodiment of the needle of the FIG. 1;
FIG. 10 is a front view of the needle of FIG. 9;
FIG. 11 is an isometric view of the needle of FIG. 9;
FIG. 12 is a side view of the setup stage of the deflected force test; and
FIG. 13 is a side view of the testing stage of the deflected force test.

### DETAILED DESCRIPTION

The Detailed Description merely describes preferred embodiments of the subject matter and is not intended to limit the scope of the subject matter or claims in any way. Indeed, the subject matter as described by the claims is broader than and unlimited by the preferred embodiments, and the terms used have their full ordinary meaning.

A medical device having a needle for performing endoscopic procedures is disclosed. The needle device is described for use with a snare in a snare injection device. However, it should be understood that this is for exemplary purposes only and the needle device may be used alone or with a variety of endoscopic devices, which are not limited to snare devices. Further, the subject matter will be described for use with an endoscope having an instrument channel. It should also be understood that this is for exemplary purposes only and the subject matter can be applied to a wide variety of applications.

In embodiments of the subject matter having a dual lumen catheter member, the needle and second endoscopic device are generally individually routed within separate lumens of a dual lumen catheter member. However, in certain embodiments, the needle may be routed within a single lumen of a single lumen catheter member. It should also be noted that a single lumen could be used for much of the device length and then the final distal length, at or about 3 to 6 inches, could employ a dual or multi-lumen catheter that is affixed to the single lumen. This can separate the two distal mechanisms so they do not become entangled, but utilizes a less complicated, less expensive, single lumen catheter for much of the length. The needle and the second endoscopic device are independently deployable by manipulation of separate control handles by one or two medical operators. In discussing the device, the terms distal and proximal are used with respect to the operator's hand. In other words, when the device is used within the auxiliary channel of an endoscope or similar device, the proximal and distal orientation are relative to the surgeon or operator of the device, and not the internal work site within the patient.

In an exemplary embodiment, the device includes a needle and a second endoscopic device, each separately routed in one lumen of a dual lumen catheter member. The needle may perform several functions during the procedure, including pre-procedure and post-procedure injections, and lifting the tissue during the procedure. The device also allows for immediate repeat injections which may be required due to absorption of the lift fluid by the gastrointestinal wall, without removal of the second endoscopic device.

The subject matter offers numerous other improvements over prior art needle devices including in a variety of endoscope configurations to prevent the needle from puncturing and wedging into the side walls of the lumen when the device is inserted into a flexible endoscope optionally configured to have an extreme tortuous pathway bent to have a small radius, such as a 1 inch radius. In certain prior art designs, a needle could injure a patient or damage the channel of the endoscope by puncturing the sidewall of a device or deploying beyond a desired length to cause harm to a patient. Meanwhile, the improvements enable the snare to freely move in and out from the flexible endoscope optionally configured to have an extreme tortuous pathway bent to have a small radius, such as a 1 inch radius. As such, the present subject matter offers improvements in patient safety and ease of use for the physician.

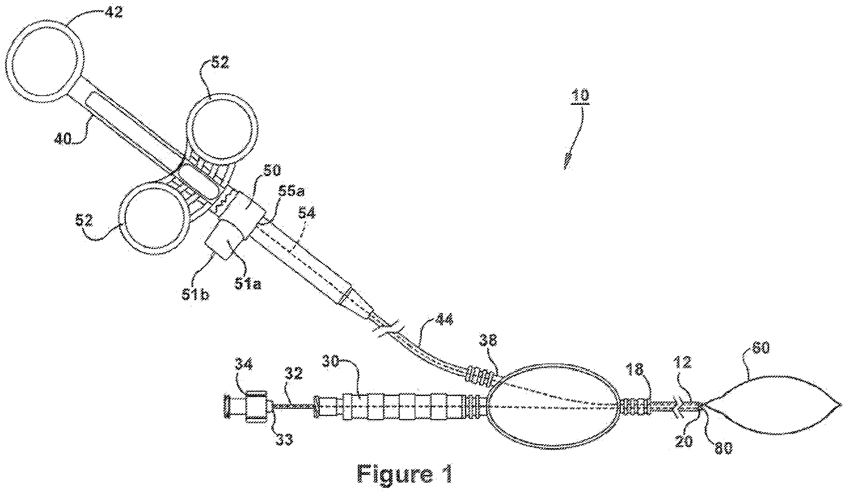

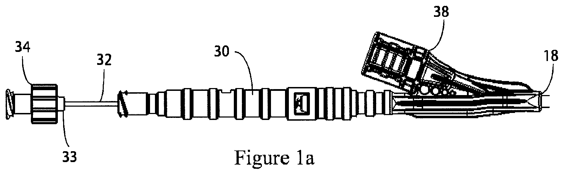

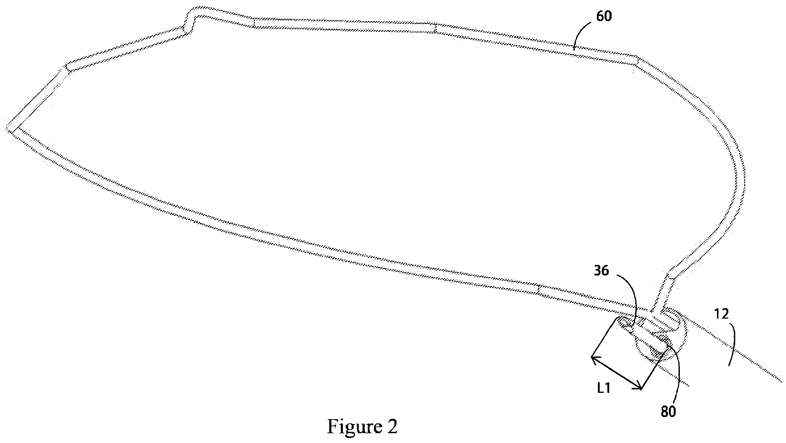

Referring now to the drawings, a snare injection device 10 for use with an endoscope is illustrated in FIG. 1. The device is suitable for use in polypectomy procedures and any other procedure in which injection and snare capability is required. In FIG. 1, the snare 60 is shown in an expanded position. As shown, the device 10 utilizes an oval snare. However, it should be apparent to those skilled in the art that various sizes and shapes of snares may be utilized effectively, for example a hexagonal snare or some other polygonal shape. FIG. 2 is an enlarged perspective view of the distal end of the device 10, showing the snare 60 expanded and the needle 36 deployed. The device allows a physician to perform either technique, i.e., operation of the snare 60 or the needle 36, without removing the device 10 from the instrument channel. Exemplary snare and needle devices are disclosed in both U.S. Patent No. 8,343,168 and U.S. Patent No. 7,691,110, and are hereby incorporated in their entirety, to the extent that they are not conflicting with the present application. In some embodiments, the needle is a standard 13 degree triple grind needle.

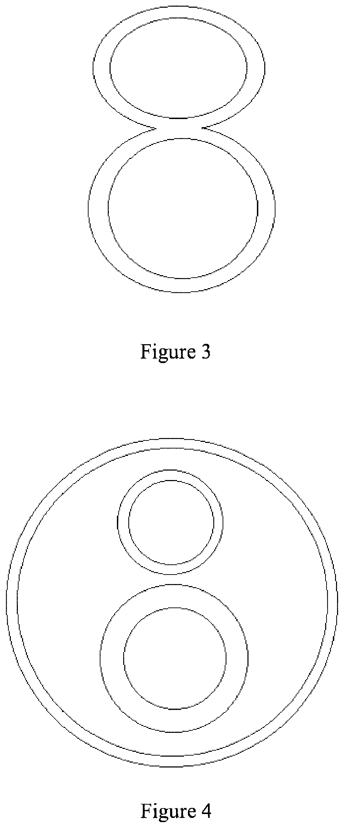

As shown in FIG. 2, the device 10 includes an elongated catheter member 12, or catheter. The depicted catheter member 12 has two lumens or channels, a first lumen (i.e., a needle lumen) and a second lumen (i.e., a snare lumen), each lumen leading from a proximal end 18 to a distal end 20 of the catheter member 12. As stated, in certain embodiments, the catheter member may have only a single lumen or channel. The catheter member 12 shown is a single piece of extruded plastic and may be constructed from a variety of flexible materials, such as for example but not limited to, polytetrafluorethylene (PTFE) or polyethylene (PE) tubing. Various first and second lumen stiffness, sizes and shapes may be used in the practice of the subject matter, as will be discussed. Further, referring to FIGS. 3 and 4, it should be apparent to one with ordinary skill in the art that the catheter member of the subject matter may be practiced with a single piece of extruded dual lumen tubing, or alternatively, with a dual lumen assembly including separate tubing and a sheath or other suitable covering. Referring to FIGS. 2, 5, and 6, the maximum distance L1 the needle protrudes is pre-determined by a needle housing 80 and the stopper 70 of the device 10. The distance L1 is determined by performance and safety criteria, and can vary in the practice of the subject matter.

A fluid delivery system for use to inject tissue within the body may be part of the device 10. The delivery system generally includes a hollow base assembly 30 (shown in FIG. 1a) fixed to a proximal end 18 of the catheter member 12. The base assembly 30 provides support for an actuator tube assembly 32 that is threaded through the base and can be moved relative thereto. The actuator tube assembly 32 has a proximal end 33 and a distal end. As shown, the tube assembly 32 is inserted through the base assembly 30 and the first lumen. The actuator tube assembly 32 can be manipulated by movement of a hollow knob 34. The knob 34 is fixed to the actuator tube assembly 32 proximal end 33. The needle 36 is fixed to the actuator tube assembly 32 distal end. In this position, a fluid solution can be passed under pressure through the knob 34 to the needle 36. To deploy the needle 36, the operator presses the knob 34 toward the base assembly 30. When the knob 34 is released, a spring mechanism (not shown) disposed internally in the base assembly 30 forces the knob 34 and needle 36 to return to their at rest position (as shown in FIG. 1). In some embodiments, the actuator tube assembly 32 may be constructed from any flexible, durable material such as polyetheretherketone (PEEK). In some embodiments, a proximal portion of the actuator tube assembly 32 is constructed from a steel hypotube or similar mechanism, and a distal portion of the actuator tube assembly 32 is constructed from any flexible, durable material such as polyetheretherketone (PEEK). In some embodiments, the actuator tube assembly 32 has an outer diameter of 0.035 inch. It should be noted that the subject matter may also be used in systems without a fluid delivery system. In these systems, the needle may be attached to an actuator, such as a cable.

The base assembly 30 further includes a side entry port 38. As shown, this port 38 is angled less than 45 degrees with respect to a longitudinal axis of the base assembly 30. It is believed this angled structure reduces binding within the dual lumen member 12. The resulting y-shaped base may be a single molded piece or a multiple part assembled piece.

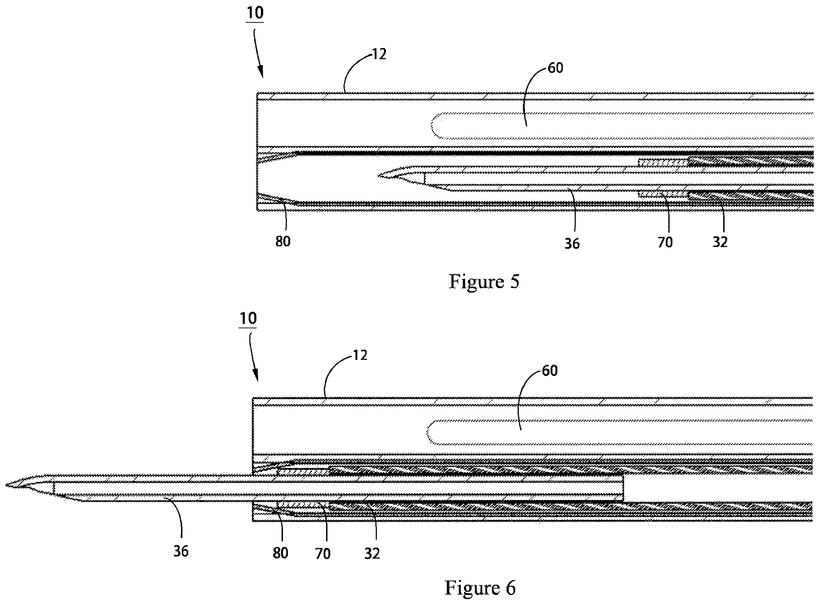

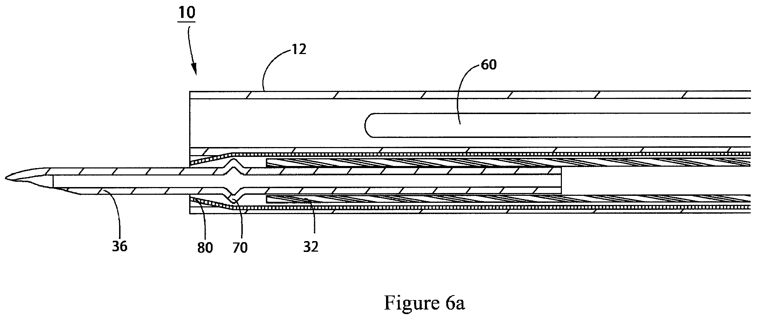

A snare system for resecting tissue is included in the device 10. The snare system can be used to remove tissue, such as for example, a polyp after it has been lifted by the injection of a fluid. The snare may be a hot snare or a cold snare. The snare 60 is shown in a collapsed or retracted position in FIGS. 5 and 6. The system includes an elongated body 40 having a thumb ring 42 at a proximal end. A handle 50 is formed on the body 40 as a separate piece. The handle is slidable relative to the body in either a distal or proximal direction by manipulation of two finger rings 52. The base 40 and handle 50 are formed of a rigid plastic material, although any suitable material may be used in the practice of the subject matter. The snare system includes a cable assembly 54, or actuator, that extends substantially through the second lumen of the catheter member 12. The cable assembly 54 has a proximal end 55a fixed to the handle 50 and a distal end fixed to a connector connecting the cable to the snare 60. Alternatively, the distal end of the cable assembly 54 is fixed to the snare 60 directly. As shown, the cable 54 further passes through a flexible tube 44 that remotely connects the body 40 to the inlet port 38. The tube 44 may be constructed from any flexible, durable material such as polyethylene or PTFE. However, this junction may also be constructed from a rigid material.

In FIGS. 5 and 6, a range of motion of the needle 36 of the device 10 is illustrated. FIG. 5 is a cross sectional view of the needle 36 retracted in a stored position within the first lumen of the catheter member 12. In this position, no axial forces are on the needle 36. FIG. 6 is a cross sectional view of the needle 36 in a fully deployed position. As shown, a stopper 70 on the needle 36 is sized to prevent the needle from deploying beyond a desired distance or falling out of the first lumen. In some embodiments, the stopper 70 is disposed on the actuator tube assembly 32. The stopper may comprise a hypotube or similar mechanism. The needle 36 may be attached to the actuator tube assembly 32 by any suitable method known in the art. For example, the needle 36 may be press fit in the distal end of the actuator tube assembly 32 or attached to the actuator tube by a connector or by adhesive or by other methods. Further, the stopper 70 and the needle 36 may be made of a unitary construction. In some embodiments, the stopper 70 and the actuator tube assembly 32 may be made of a unitary construction. When the needle 36 is at the stored position, the needle 36 rests a predetermined distance back in the first lumen. In some embodiments, a needle integrates the stop. This can be accomplished by a physical upset/deformation of the needle or by manufacturing it by metal injection molding or ceramic or polymer molding so that portion of the geometry is already present. In addition, the actuator tube could be flared or contain an integral bump, that could act as the stop to control maximum needle projection, as shown in FIG. 6a.

As shown in FIGS. 5 and 6, the needle 36 is disposed within a needle housing member 80 attached to the first lumen. The distal end of the housing member 80 is tapered and does not extend beyond the distal end of the first lumen. Alternatively, the distal end of the housing member 80 extends beyond the distal end of the first lumen. In some embodiments, the distal end of the housing member 80 comprises a stop that is not tapered. In another embodiment, the distal end of the housing member 80 and the distal end of the first lumen are even. The housing member 80 provides a guide for the needle assembly, limits the travel of the needle, and assists to prevent the needle from puncturing the lumen, wedging into the side walls, or falling out of the catheter. The housing member 80 may be disposed in place by being heat shrunk, press fit, bonded, or other suitable known method. The housing member 80 may also have barbs, or ribbed sections, that create a friction surface against the first lumen to hold the housing member in place. The housing member 80 has a predetermined length so that the needle 36 is disposed within the housing member 80 when the needle 36 is at the stored position. The housing member 80 may be constructed from any coated metal or non-conductive suitable material, such as for example, a medium hardness plastic. A benefit of the housing member 80 being non-conductive is that radio frequency energy used to energize the snare 60 does not transfer to the housing member which in turn would direct the current away from the targeted tissue. The housing member 80 may include two or more pieces joined together. The housing member 80 may be a spring sheath.

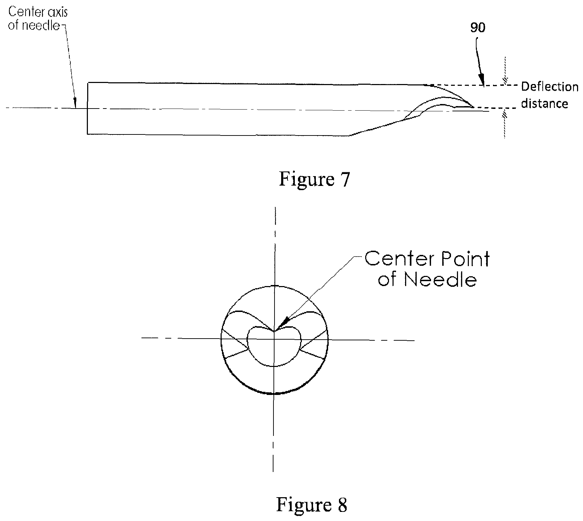

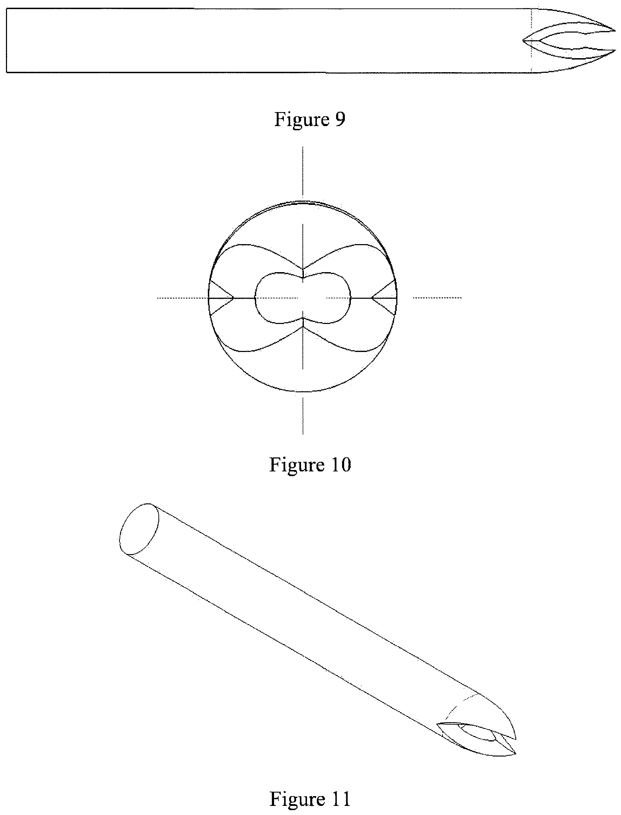

One aspect of the present subject matter is a deflected, centered, or oriented needle tip. As shown in an embodiment of FIGS. 7 and 8, the needle tip is deflected towards the center axis of the needle. In some embodiments, the deflection 90 of the needle is between 0.001 inch and 0.1 inch. The deflection of the needle is between 0.0016 inch and 0.0114 inch. As shown in another embodiment of FIGS. 9-11, the needle is a two-point deflected needle design.

Another aspect of the present subject matter is a dual-lumen catheter member providing stiffness to the first lumen (i.e., the needle lumen) to help with the needle deployment, and flexibility to the second lumen (i.e., the snare lumen). The stiff needle lumen provides more support for the needle, which helps to keep the needle straight so it can be deployed without puncturing the lumen. The snare lumen can be more flexible so that the snare can easily deploy in tortuous configurations. In some embodiments, the deflected force of the needle lumen is 1 - 3 times as strong as the snare lumen. In some embodiments, the deflected force of the needle lumen is 1.7 times as strong as the snare lumen.

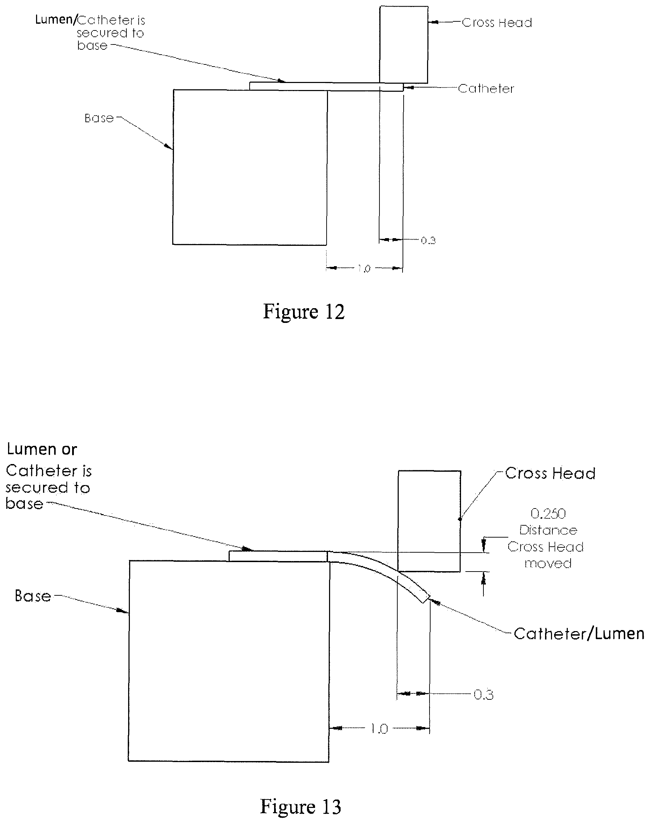

The deflected force of the lumens or catheter is defined by the test shown in FIGS. 12 and 13 that hanging a lumen or catheter member 12 one inch off an edge of the testing base and measuring the force required to deflect on the catheter member. The testing setup for measuring the stiffness of the catheter member comprises 1) crosshead moved at 1 inch per minute; 2) crosshead moved 0.25 inch; 3) 5 lbs load cell was used; and 4) the catheter member is 1 inch from the edge of the testing base.

| Deflected Force Measurement for Individual Catheter Lumens (lbf) | |
|---|---|
| Needle Lumen | Snare Lumen |
| 0.113 | 0.069 |
| 0.134 | 0.067 |
| 0.130 | 0.067 |
| 0.126 | 0.066 |
| 0.117 | 0.065 |
| Average Force = 0.12 | Average Force = 0.07 |

In addition, the overall catheter member 12 (without a needle or a snare) needs to have a specific stiffness so that it can pass through an extreme tortuous pathway, such as a 1 inch radius in the endoscope. Therefore, the same test can be performed with varying inserts in the distal end of the catheter member. It was determined that the force to push down an overall catheter that is hanging 1 inch off the edge of a testing base should be between 0.17 lbf and 0.27 lbf. In some embodiments, the force to push down an overall catheter that is hanging 1 inch off the edge of a testing base should be between 0.16 lbf and 0.30 lbf.

| Deflected Force Measurements for Catheter Members with varying inserts (lbf) | | | |
|---|---|---|---|
| | Only Catheter without a needle housing member | Catheter with a PEEK needle housing member | Catheter with a Spring Sheath needle housing member |
| Trial 1 | 0.161 | 0.216 | 0.252 |
| Trial 2 | 0.169 | 0.285 | 0.297 |
| Trial 3 | 0.174 | 0.207 | 0.276 |
| Trial 4 | 0.169 | 0.210 | 0.265 |
| Trial 5 | 0.165 | 0.239 | 0.258 |
| Average | 0.170 | 0.230 | 0.270 |
| Can it pass through an extreme tortuous pathway, such as a 1 inch radius of a 2.8 mm endoscopic instrument channel? | Yes | Yes | Yes |

While several embodiments of the invention have been illustrated and described in considerable detail, the present invention is not to be considered limited to the precise construction disclosed. Various adaptations, modifications and uses of the invention may occur to those skilled in the arts to which the invention relates. It is the intention to cover all such adaptations, modifications and uses falling within the scope or spirit of the claims filed herewith.

## Claims

1. A medical device for performing an endoscopic procedure through an endoscope, the device comprising:
a catheter 12 having a first lumen and a second lumen;
a needle system for injecting a target tissue within a body, wherein the needle system comprises a needle 36, and an actuator tube 32, wherein the needle is connected to a distal end of the actuator tube; and
a base 30 disposed at a proximal end 18 of the catheter, wherein the actuator tube is inserted through the base and the first lumen; the needle operably protrudes a predetermined distance out of a distal end 20 of the catheter.

2. The medical device of claim 1, wherein the needle system further comprises a needle housing member, positioned between the needle and the first lumen when the needle is in a stored position, wherein the needle housing member has a predetermined length so that the needle is disposed within the needle housing member when the needle is at the stored position, the needle housing member has a continuous inner surface and prevents the needle from penetrating the first lumen.

3. The medical device of claim 2, wherein a distal end of the needle housing member is tapered and does not extend beyond a distal end of the first lumen.

4. The medical device of claim 2, wherein a distal end of the needle housing member limits the length of the needle protruding out of the distal end of the catheter.

5. The medical device of claim 3, wherein the distal end of the needle housing member and the distal end of the first lumen are even.

6. The medical device of claim 2, wherein the needle housing member comprises barbs or ribbed sections against the first lumen to hold the housing member in place.

7. The medical device of claim 2, wherein the needle system further comprises a stopper, sized and configured to prevent the needle from deploying beyond a desired distance or falling out of the first lumen.

8. The medical device of claim 2, wherein the actuator tube is selectively manipulated by movement of a hollow knob 34, wherein the knob is fixed to a proximal end of the actuator tube.

9. The medical device of claim 2, wherein the catheter further comprises a covering tube, and the first and second lumens are disposed through the covering tube.

10. The medical device of claim 2 further comprising a snare system for resecting the targeted tissue, wherein the snare system comprises a snare 60, and an actuating cable, wherein the cable is inserted through the second lumen.

11. The medical device of claim 1, wherein the needle comprises a tip, oriented towards a center axis of the needle.

12. A medical device according to any one of the preceding claims, wherein the needle is a two-point deflected needle.

13. A medical device according to any one of the preceding claims, wherein the first lumen and second lumen have different deflected forces.

14. The medical device of claim 13, wherein the deflected force of the first lumen is larger than the deflected force of the second lumen.

15. The medical device of claim 14, wherein the deflected force of the first lumen is about 1-3 times as strong as the second lumen.
